(19) Europäisches Patentamt
European Patent Office
Office européen des brevets

(11) Veröffentlichungsnummer: **0 025 939**
**B1**

(12) # EUROPÄISCHE PATENTSCHRIFT

(45) Veröffentlichungstag der Patentschrift: **26.05.82**

(51) Int. Cl.³: **C 07 C 121/34,** C 07 C 120/00, A 61 K 7/46

(21) Anmeldenummer: **80105406.5**

(22) Anmeldetag: **10.09.80**

(54) **Aliphatische Methoxy- und Hydroxynitrile, deren Herstellung und deren Verwendung als Riechstoff.**

(30) Priorität: **25.09.79 DE 2938689**

(43) Veröffentlichungstag der Anmeldung: **01.04.81 Patentblatt 81/13**

(45) Bekanntmachung des Hinweises auf die Patenterteilung: **26.05.82 Patentblatt 82/21**

(84) Benannte Vertragsstaaten: **BE CH DE FR GB LI NL**

(56) Entgegenhaltungen:
**DE-A-2 333 265**
**DE-A-2 348 359**
**DE-A-2 548 420**
**DE-A-2 548 502**
**GB-A-1 005 897**

(73) Patentinhaber: **BASF Aktiengesellschaft, Carl-Bosch-Strasse 38, D-6700 Ludwigshafen (DE)**

(72) Erfinder: **Thoemel, Frank, Dr., Leberstrasse 17, D-6940 Weinheim (DE)**
Erfinder: **Hoffmann, Werner, Dr., Ringstrasse 11 C, D-6701 Neuhofen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



EP 0 025 939 B1

# Aliphatische Methoxy- und Hydroxynitrile, deren Herstellung und deren Verwendung als Riechstoff

Die vorliegende Erfindung betrifft gesättigte oder einfach ungesättigte aliphatische Methoxy- und Hydroxynitrile der allgemeinen Formel I

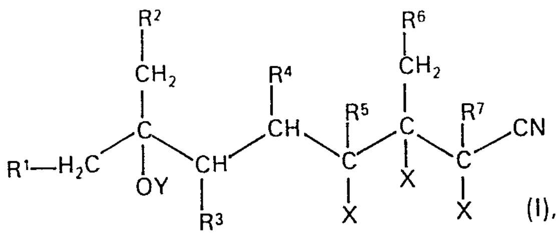

(I),

in der $R^1$ bis $R^7$ und Y für H oder $-CH_3$ stehen und die X entweder alle H bedeuten oder jeweils 2 benachbarte X für eine weitere Bindung zwischen den sie tragenden C-Atomen stehen und das dritte X -H bedeutet, Verfahren zu deren Herstellung und deren Verwendung als Riechstoff.

Die neuen Methoxy- und Hydroxynitrile weisen interessante, vorwiegend fruchtige bis blumige Geruchsnoten auf und erweitern dadurch die Palette wertvoller vollsynthetischer Duftstoffe.

Es war bekannt, ungesättigte aliphatische Nitrile als Riechstoffe zu verwenden. So werden beispielsweise in der US-PS 3,655,722 monoterpenoide Nitrile, wie cis-3,7-Dimethyl-2,6-octadiennitril (Geranonitril) und trans-3,7-Dimethyl-2,6-octadiennitril und deren Verwendung als Riechstoffe beschrieben.

Auch einige Hydroxynitrile mit Riechstoffeigenschaften sind bekannt. So wurden 3-Hydroxy-nitrile durch Aldoladdition von Acetonitril und Ketonen hergestellt (vgl. DE-OS 2548420). Die DE-OS 2333265 beschreibt die Hydratisierung von 1-Methyl-4-(1-cyano-propen-2-yl)-cyclohex-1-en durch 53%ige Schwefelsäure bei 15°C:

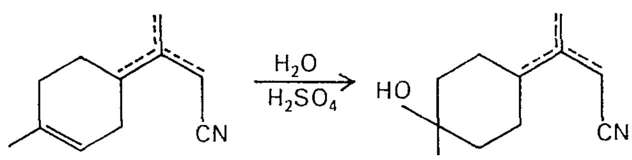


In der DE-OS 2635545 ist vermerkt, dass «Geranonitril bei längerer Behandlung bei Raumtemperatur mit einer Mischung aus 98%iger Schwefelsäure und Eisessig in Volumenteilen von 5 bis 25 bei Zugabe von Wasser zu wenig flüchtigen teerartigen Substanzen führt», während das in 6-Stellung hydrierte 3,7-Dimethyl-2-octen-nitril unverändert bleibt.

Es ist ebenfalls bekannt, dass sich Cyanessigsäureäthylester und 2-Hydroxy-2-methyl-heptan-6-on in Gegenwart von Wasserstoff und Ammoniumacetat/Essigsäure in Äthanol zu 2-Cyano-7-hydroxy-3,7-dimethyl-octansäureäthylester umsetzen lassen (DE-OS 2333265).

Die nach alkalischer Verseifung erhaltene Hydroxysäure wurde pyrolisiert, wobei neben der Decarboxylierung auch eine Dehydratisierung unter Bildung des ungesättigten Nitrils eintrat:

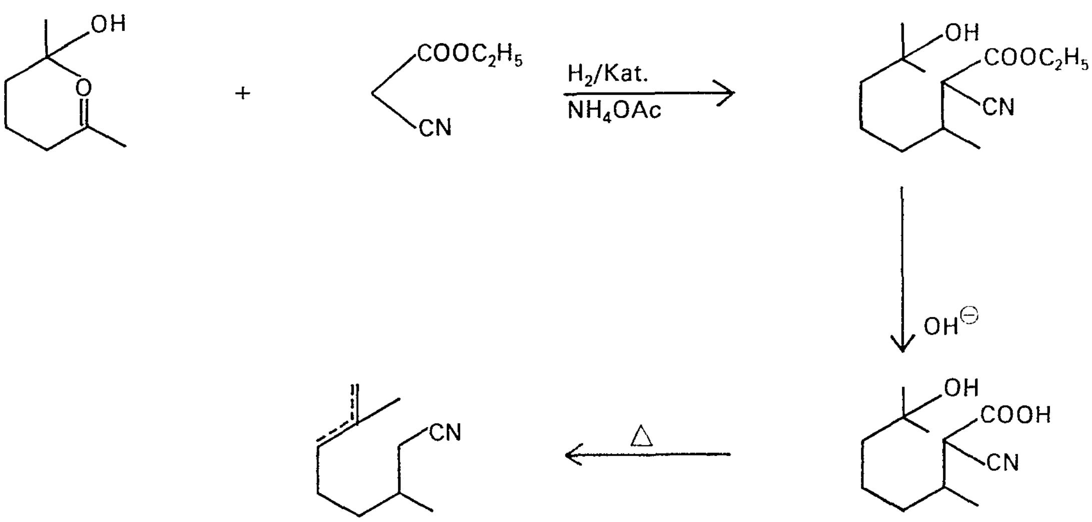


Es wurde nun gefunden, dass ungesättigte Nitrile der allgemeinen Formel II mit Hilfe von Mineralsäuren, insbesondere von verdünnter Schwefelsäure, hydratisiert werden können, was aufgrund der oben zitierten Literatur nicht ohne weiteres zu erwarten war. Bei der Hydratisierung bildeten sich die erfindungsgemässen Hydroxynitrile mit ihren interessanten fruchtigen bis blumigen Duftnoten. Überraschenderweise weisen sie in der Regel nicht den Nitrilen oft eigenen störenden Charakter auf. Sie riechen für Hydroxyverbindungen verhältnissmässig stark und haben zudem in

Riechstoffkompositionen eine fixierende Wirkung.

Gegenstand der Erfindung ist neben den Methoxy- und Hydroxynitrilen der Formel I auch ein Verfahren zur Herstellung der aliphatischen Hydroxynitrile der allgemeinen Formel I, das dadurch gekennzeichnet ist, dass man die entsprechenden Nitrile der allgemeinen Formel II

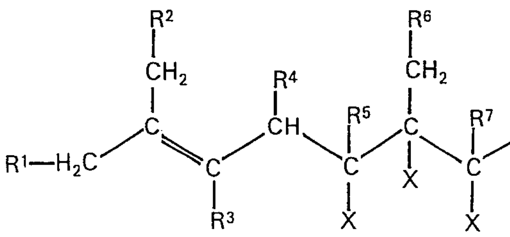

(II),

in der $R^1$ bis $R^7$ und die X die oben angegebene Bedeutung haben, durch Behandeln mit verdünnten Mineralsäuren hydratisiert und gegebenenfalls anschliessend katalytisch hydriert.

Gegenstand der Erfindung ist weiterhin ein Verfahren zur Herstellung der aliphatischen Methoxy- oder Hydroxinitrile der allgemeinen Formel I, in der $R^7$ für -H steht, das dadurch gekennzeichnet ist, dass man die entspechenden Methoxy- bzw. Hydroxyketone der allgemeinen Formel III

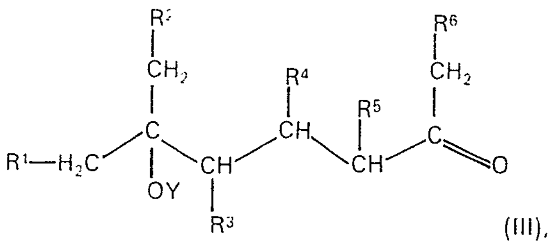

(III),

in der $R^1$ bis $R^6$ und Y die in Anspruch 1 angegebenen Bedeutung haben, in Gegenwart eines schwachbasischen Katalysators mit Cyanessigsäure umsetzt, wobei das sich bildende Kondensationsprodukt dercarboxyliert wird, und gegebenenfalls anschliessend katalytisch hydriert.

Die als Ausgangsstoff für die erfindungsgemässen Verfahren benötigten Nitrile der Formel II sind bekannte, handelsübliche Verbindungen. Die Hydroxyketone der Formel III können beispielsweise durch Hydratisierung von entsprechend alkylsubstituierten Hept-5-en-2-onen in Gegenwart von verdünnter Schwefelsäure gewonnen werden. Bei Zusatz von Methanol erhält man die entsprechenden Methoxyalkylheptan-2-one.

Zur Durchführung der erfindungsgemässen Hydratisierung rührt man die in 6-Stellung bzw. die in 2- und 6-Stellung ungesättigten Nitrile in der Regel bei Temperaturen von -20 bis +50°C, vorzugsweise -10 bis +25°C mit einer verdünnten wässrigen Mineralsäure, vorzugsweise einer 20 bis 75, insbesondere einer 45 bis 55 gewichtsprozentigen Schwefelsäure. Auch wässrige Phosphorsäure, Salzsäure oder eine wässrige Suspension saurer Jonenaustauscher können für das Hydratisieren eingesetzt werden.

Die Umsetzung kann dünnschichtchromatographisch verfolgt werden. Die Reaktionsbedingung für die einzelnen Nitrile weichen ein wenig voneinander ab. Nach vollständiger oder weitgehender Umsetzung der Reaktionspartner wird das Reaktionsgemisch mittels einer Base, wie etwa 50%iger Natronlauge auf einen pH-Wert von 6 gebracht, mit einem wasserschwerlöslichen Lösungsmittel, wie Diäthyläther, Toluol oder Chloroform extrahiert und die gewaschenen Extrakte destillativ aufgearbeitet.

Die Kondensation der Methoxy- bzw. Hydroxyketone der Formel III mit Cyanessigsäure erfolgt in an sich bekannter Weise (vgl. DE-OS 2 348 359). Als schwach basische Katalysatoren können für das Verfahren z. B. Ammoniak, Ammoniumsalze, primäre oder sekundäre Amine, Pyridin, Piperidin oder Chinolin, vorzugsweise Ammoniumacetat, Pyridin und Piperidin verwendet werden.

Die Umsetzung wird zweckmässig in einem organischen Lösungsmittel durchgeführt. Als Lösungsmittel werden mit Vorteil solche verwendet, die mit Wasser azeotrope Gemische bilden, wie Benzol, Toluol oder Xylol.

Die Reaktionstemperatur für diese Kondensation kann im allgemeinen zwischen ca. 20 und 180°C variieren. Zweckmässigerweise führt man die Reaktion im Siedebereich des jeweils verwendeten Lösungsmittels durch. Besonders vorteilhaft ist es, das bei der Kondensation gebildete Wasser mit dem Lösungsmittel azeotrop abzutrennen.

Das Keton der Formel III und Cyanessigsäure werden im allgemeinen in etwa äquimolarer Menge eingesetzt, jedoch kann auch die jeweils billigere Komponente in einem leichten Überschuss verwendet werden.

Der schwach basische Katalysator wird im allgemeinen in Mengen von 0,01 bis 2 Mol, bezogen auf 1 Mol Cyanessigsäure, eingesetzt werden.

Nach der Abscheidung des bei der Reaktion gebildeten Wassers wird das Lösungsmittel und gegebenenfalls auch der schwach basische Katalysator abdestilliert. Aus dem Rückstand werden die erfindungsgemässen ungesättigten Nitrile durch fraktionierte Destillation unter vermindertem Druck rein erhalten.

Das Verhältnis der nach diesem Prozess erhaltenen in 2-Stellung ungesättigten zu den in 3-Stellung ungesättigten Hydroxynitrilen kann der als Katalysator verwendeten Base beeinflusst werden (vgl. Beispiel 10 und 11).

Die ungesättigten Methoxy- und Hydroxynitrile der Formel I lassen sich in an sich bekannter Weise katalytisch zu den gesättigten Methoxy- bzw. Hydroxynitrilen der Formel I hydrieren. Die Hydrierung erfolgt mit Vorteil in Gegenwart von Pd-Katalysatoren, insbesondere von Katalysatoren bestehend aus Pd auf Aktivkohle. Die Reaktionstemperatur beträgt im allgemeinen 0 bis 100°C, der $H_2$-Druck etwa 1 bis 10 bar.

Die erfindungsgemässen Methoxy- bzw. Hydroxynitrile finden Verwendung als Duftstoffe in kosmetischen Zubereitungen aller Art sowie in

Wasch- und Reinigungsmitteln, als Duftverbesserer für Leime, Anstriche und ähnliche Erzeugnisse oder als Fixateure in Riechstoffkompositionen.

Besonders ansprechende Duftnoten besitzen: 7-Hydroxy-3,7-dimethyl-2(3)-octennitril, 7-Hydroxy-2,3,7-trimethyl-6-octannitril und 7-Methoxy-3,7-dimethylo-octannitril.

Beispiel 1 bis 6

Herstellung von 7-Hydroxy-3,7-dimethyl-octannitril

Zu der in der folgenden Tabelle angegebenen Menge an Schwefelsäure der angegebenen Konzentration wurde bei der für jedes Beispiel angegebenen Reaktionstemperatur innerhalb von 5 Minuten die jeweils angegebene Menge an 3,7-Dimethyl-6-octen-nitril addiert. Anschliessend liess man das Reaktionsgemisch unter Rühren bis zu möglichst vollständigem Umsatz bei der Reaktionstemperatur weiterreagieren (Umsatzkontrolle: dünnschichtchromatographisch auf Kieselgel-Fertigplatten der Firma Merck mit Cyclohexan/Essigsäureäthylester (3/1) als Laufmittel und $KMnO_4$ in konz. $H_2SO_4$ als Sprühreagenz). Anschliessend wurde das Reaktionsgemisch mit 50%iger wässriger NaOH unter Kühlung auf einen pH-Wert von 6 eingestellt und mit Diäthyläther extrahiert. Die Extrakte wurden mit Wasser neutralgewaschen, getrocknet und unter vermindertem Druck destilliert. Die jeweils erzielten Umsätze an Ausgangsnitril und Ausbeuten an 7-Hydroxy-3,7-dimethyl-octannitril sind aus der folgenden Tabelle ersichtlich.

Physikalische Daten:

Kp = 85-86°C bei 0,1 mbar; $n_D^{20}$ = 1,4555;
IR-Spektrum (in Substanz): 3420 (-OH), 2238 (-CN), 1460, 1370, 1150 $cm^{-1}$;
$^1$HNMR-Spektrum ($CDCl_3$, 100 MHz):
δ = 1,1 (d, 3 H, $-CH_3$), 1,2 (s, 6 H, $-CH_3$), 1,4 (s, 6 H, $-CH_2-$), 1,8 (m, 1 H, -CH), 2,15 (s, 1 H, -OH), 2,28 ppm (d, 2 H, $-CH_2$);
Duftnote: blumig, Richtung Rose, krautig.

| Beispiel | $H_2SO_4$ | | | Nitril | | Reaktionstemperatur [°C] | Reaktionszeit | Umsatz | Ausbeute⁺ |
|---|---|---|---|---|---|---|---|---|---|
| | [g] | [Gew.-%] | [mol] | [g] | [mol] | | [h] | [%] | [%] |
| 1 | 260 | 50 | 1,33 | 75,5 | 0,5 | 20 | 4 | 100 | 56 |
| 2 | 1560 | 50 | 7,96 | 453 | 3 | 20 | 4 | 89 | 45 |
| 3 | 104 | 50 | 0,54 | 30,2 | 0,2 | 20 | 8 | 100 | 60 |
| 4 | 104 | 50 | 0,54 | 30,2 | 0,2 | 0 | 8 | 75 | 93 |
| 5 | 104 | 25 | 0,27 | 30,2 | 0,2 | 50 | 5 | 34 | 50 |
| 6 | 104 | 50 | 0,54 | 30,2 | 0,2 | 40 | 1 | 99 | 13,6 |

⁺ Ausbeute, bezogen auf umgesetztes Nitril

Beispiel 7

Analog Beispiel 1 wurden 49 g (0,32 mol) 2,3,7-Trimethyl-6-octen-nitril und 163 g (0,83 mol) einer 50 gew.-%igen Schwefelsäure bei 20°C umgesetzt. Nach 13 Stunden wurde die Reaktion abgebrochen und das Reaktionsgemisch analog Beispiel 1 aufgearbeitet. Es wurden 16,4 g nicht umgesetztes Nitril zurückgewonnen. Die Ausbeute an 7-Hydroxy-2,3,7-trimethyl-octannitril betrug 17,2 g, entsprechen 48%, bezogen auf umgesetztes 2,3,7-Trimethyl-6-octennitril.

Physikalische Daten:

Kp = 96 - 98°C bei 0,1 mbar; $n_D^{20}$ = 1,4529;
$^1$HNMR-Spektrum ($CDCl_3$, 100 MHz):
δ = 1,05 (d, 3 H, $-CH_3$), 1,21 (s, 6 H, $-CH_3$), 1,26 (d, 3 H, $-CH_3$), 1,41 (s breit, 6 H, $-CH_2$), 1,62 (m, 1 H, -CH); 1,78 (s, 1 H, -OH), 2,6 ppm (m, 1 H, -CH);
Duftnote: frisch, krautig-holzig, fruchtig, leicht citronenartig.

Beispiel 8

149 g (1 mol) 3,7-Dimethyl-2,6-octadiennitril (Geranonitril) wurden bei Raumtemperatur unter Rühren innerhalb von 30 Minuten zu 525 g (2,4 mol) einer 50 gew.-%igen Schwefelsäure addiert. Anschliessend liess man das Reaktionsgemisch unter Rühren über Nacht nachreagieren und arbeitete es dann analog Beispiel 1 auf.

Die Ausbeute an 7-Hydroxy-3,7-dimethyl-2-octennitril betrug 95,2 g (57% d. Th.).

Physikalische Daten:

E/Z-Verhältnis ca. 65:35;
Kp = 102-104°C bei 0,3 mbar; $n_D^{20}$ = 1,4742;
$^1$HNMR-Spektrum ($CDCl_3$, 100 MHz):
δ = 1,18 (s, 6 H, $-CH_3$), 1,48 (m, 4 H, $-CH_2$), 1,88 (s, 1,05 H, $-CH_3$ [Z]), 2,0 (s, 1,95 H, $CH_3$ [E]), 2,25 (m, 2 H, $-CH_2$), 5,1 pm (s, 1 H, = CH);
Duftnote: stark, citronenartig.

Beispiel 9

Analog Beispiel 1 wurden 76,5 g (0,5 mol) 3,7-Dimethyl-2,6-nonadien-nitril und 434 g (3,1 mol) einer 70 gew.-%igen Schwefelsäure 1 Stunde lang bei -10°C umgesetzt. Der Umsatz an 3,7-Dimethyl-2,6-nonadiennitril betrug 94%. Die Ausbeute an 7-Hydroxy-2,7-dimethyl-2-nonennitril betrug 47,6 g (entsprechend 62% d. Th. bzw. 66%, bezogen auf umgesetztes Nitril).

Physikalische Daten:

Kp = 102-105°C bei 0,1 mbar;
$n_D^{20}$ = 1,4792;
$^1$HNMR-Spektrum ($CDCl_3$, 100 MHz):
δ = 0,9 (t, 3 H, $-CH_3$), 1,15 (s, 3 H, $CH_3$), 1,2-1,8 (m, 7 H, $-CH_2$, -OH), 1,9 (s, 1,4 H, $-CH_3$ [Z]), 2,03 (s, 1,6

H, $-CH_3$ [E]), 2,0–2,5 (m, 2 H, $-CH_2$), 5,1 ppm (s, 1 H, =CH);
Duftnote: stark, citronenartig, blumig.

Beispiel 10

Ein Gemisch bestehend aus 192 g (1,5 mol) 6-Hydroxy-6-methyl-heptanon-(2), 128 g (1,5 mol) Cyanessigsäure, 150 g (1,5 mol) Cyclohexylamin, 0,5 mol Hydrochinon und 500 ml Toluol wurde 5 Stunden unter Rückfluss zum Sieden erhitzt, wobei das entstehende Wasser (27 g) azeotrop abdestilliert wurde. Das erhaltene Reaktionsgemisch wurde mit Wasser und dann mit 10%iger Schwefelsäure bis zur leicht sauren Reaktion und anschliessend mit 10%iger Natronlauge und Wasser neutral gewaschen und dann unter vermindertem Druck destilliert. Die Ausbeute betrug 78,9 g (31% d.Th.) eines Gemisches bestehend zu ca. 60% aus 7-Hydroxy-3,7-dimethyl-2-octen-nitril (E/Z 60 : 40) und ca. 40% aus 7-Hydroxy-3,7-dimethyl-3-octen-nitril.

Physikalische Daten:

Kp = 98–99°C bei 0,2 mbar;
$n_D^{20}$ = 1,4720;
$^1$H-NMR-Spektrum ($CDCl_3$, 270 MHz):
Anteil 7-Hydroxy-3,7-dimethyl-2-octen-nitril:
$\delta$ = 1,25 (s, $-CH_3$), 1,54 (m, $-CH_2$), 1,95 (s, $-CH_3$ [Z]), 2,19 (s, $CH_3$ [E]: 2,1–2,3 (m, $-CH_2$, -OH), 5,15 ppm (s, $-CH_3$).
Anteil 7-Hydroxy-3,7-dimethyl-3-octen-nitril:
$\delta$ = 1,27 (s, $-CH_3$), 1,54 (m, $-CH_2$), 1,54 (m, $-CH_2$), 1,78 (s, $-CH_3$), 2,1–2,5 (m, $-CH_2$, -OH), 3,05 (s, $-CH_2$), 5,5 (breites s, =CH);
Duftnote: stark, fruchtig, mandarinenartig, citronenartig.

Beispiel 11

Ein Gemisch bestehend aus 288 g (2 mol) 6-Hydroxy-6-methyl-heptanon-(2), 187 g (2,2 mol) Cyanessigsäure, 188 g Pyridin, 6,6 g Ammoniumacetat und 300 ml Toluol wurde 6 Stunden unter Rückfluss zum Sieden erhitzt, wobei 37 g Wasser azeotrop abdestilliert wurden. Anschliessend wurde aus dem Reaktionsgemisch ein Gemisch von 490 g Toluol und Pyridin abdestilliert, eine Lösung von 30 g Natriummethylat in 540 ml Methanol addiert und das Ganze noch 3 Stunden bei Raumtemperatur gerührt. Danach wurde das Reaktionsgemisch durch Zusatz von 24 g Eisessig neutralgestellt, destillativ weitgehend von Methanol befreit und auf 100 ml Eiswasser gegossen. Die organische Phase wurde abgetrennt und destilliert.

Die Ausbeute betrug 185 g (56% d.Th.) an 7-Hydroxy-3,7-dimethyl-2-octen-nitril (E/Z ca. 60:40).

Physikalische Daten:

Kp = 81–82°C bei 0,05 mbar; $n_D^{20}$ = 1,4712;
$^1$HNMR ($CDCl_3$, 80 MHz) $\delta$ = 1,2 (s, 6 H, $-CH_3$), 1,5 (m, 4 H, $-CH_2$), 1,9 und 2,01 (s, 3 H, $-CH_3$), 2,3 (s, 1 H, -OH), 1,8–2,5 (m, 2 H, $-CH_2$), 5,1 ppm (s, 1 H, = CH);
Duftnote: citronenartig.

Beispiel 12

33,4 g (0,2 mol) eines 6:4-Gemisches von 7-Hydroxy-3,7-dimethyl-2-octen-nitril und 7-Hydroxy-3,7-dimethyl-3-octen-nitril wurden in Gegenwart von 0,5 g Palladium auf Aktivkohle (5 Gew.-% Pd) bei 50°C unter Normaldruck hydriert. Nach Abfiltrieren des Katalysators erhält man durch Destillation 29 g (86%) 7-Hydroxy-3,7-dimethyl-octannitril.

Physikalische Daten:

Kp = 85–86°C, $n_D^{20}$ = 1,4556;
Duftnote: Geruch angenehm fruchtig (Himbeere), dann holzig, blumig (Richtung Hydroxycitronellal).

Beispiel 13

Ein Gemisch bestehend aus 466 g (2,95 mol) 6-Methoxy-6-methyl-heptanon-(2), 271 g (3,19 mol) Cyanessigsäure, 270 g (3,42 mol) Pyridin, 10 g Ammoniumacetat und 750 ml Toluol wurde 9 Stunden unter Rückfluss zum Sieden erhitzt, wobei sich das bildende Wasser (51 g) azeotrop abdestilliert wurde. Danach wurden bei 70–100°C Badtemperatur und 20–100 mbar ca. 900 g eines Toluol/Pyridin-Gemisches abdestilliert und der Rückstand 3 Stunden mit 850 ml einer 1 n-Natronlauge bei Raumtemperatur gerührt. Zu dem homogenen Gemisch wurde anschliessend 500 ml einer 10%igen Schwefelsäure zugeführt, die organische Phase abgetrennt und die wässrige Phase zweimal mit 30 ml Äther extrahiert. Die vereinigten organischen Phasen wurden mit verd. Natriumcarbonatlösung neutralgewaschen und die Lösungsmittel unter vermindertem Druck entfernt. Durch Destillation des Rückstandes (464 g) erhielt man 287 g (54% d.Th., 73% bezogen auf umgesetztes 6-Methoxy-6-methyl-heptanon) eines Gemisches bestehend aus ca. 60% 7-Methoxy-3,7-dimethyl-3-octennitril und ca. 40% 7-Methoxy-3,7-dimethyl-2-octennitril.

Physikalische Daten:

Kp. 61–62°C bei 0,1 mbar; $n_D^{20}$ = 1,4562;
Duftnote: citronenartig, nitrilartig $^1$H-NMR-Spektrum ($CDCl_3$, 100 MHz):
Anteil 7-Methoxy-3,7-dimethyl-3-octen-nitril (E/Z): $\delta$ = 1,13 (s, $-CH_3$), 1,44 (m, $-CH_2$), 1,55 (s, $-CH_3$[Z]) und 1,7 (s, $-CH_3$[E]), 2,0 (m, $-CH_2$), 2,97 (s, $-CH_2$[E]) und 3,04 (s, $-CH_2$[Z]), 3,1 ppm (s, $-CH_3$);
Anteil 7-Methoxy-3,7-dimethyl-2-octen-nitril: $\delta$ = 1,13 (s, $-CH_3$), 1,44 (m, $-CH_2$), 2,0 (s, $-CH_3$), 2,02 (m, $-CH_2$), 3,1 ppm (s, $-CH_3$).

Beispiel 14

95 g (0,5 mol) 7-Methoxy-3,7-dimethyl-2(3)-octennitril wurden in 100 ml Methanol gelöst und in Gegenwart von 3 g Palladium auf Calciumcarbonat (5 Gew.-% Pd) bei 50°C unter Normaldruck hydriert, bis kein Wasserstoff mehr aufgenommen wurde. Nach Abfiltrieren vom Katalysator erhielt man durch Destillation (Drehbandkolonne) 40,6 g (51% d.Th.) 7-Methoxy-3,7-dimethyl-octannitril neben 16,6 g Ausgangsmaterial.

Physikalische Daten:

Kp. 89-91°C bei 0,2 mbar, $n_D^{20} = 1,4444$;
$^1$H-NMR-Spektrum ($CDCl_3$, 220 MHz):
$\delta$ = 1,05 (d, $-CH_3$), 1,1 (s, $-CH_3$), 1,2–2,4 (m, $-CH_2$), 3,1 ppm (s, $-CH_3$);
Duftnote: blumig, fruchtig-süss, tabakartig.

**Patentansprüche**

1. Gesättigte oder einfach ungesättigte aliphatische Methoxy- und Hydroxynitrile der allgemeinen Formel I

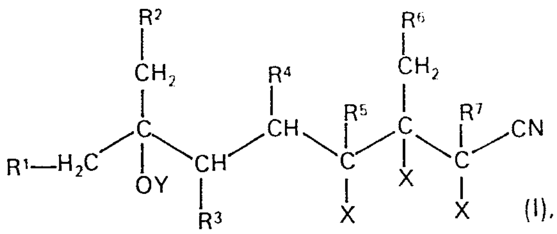

(I),

in der $R^1$ bis $R^7$ und Y für H oder $-CH_3$ stehen und die X entweder alle H bedeuten oder jeweils 2 benachbarte X für eine weitere Bindung zwischen den sie tragenden C-Atomen stehen und das dritte X-H bedeutet.

2. Verfahren zur Herstellung der aliphatischen Hydroxynitrile der allgemeinen Formel I gemäss Anspruch 1, dadurch gekennzeichnet, dass man die entsprechenden Nitrile der allgemeinen Formel II

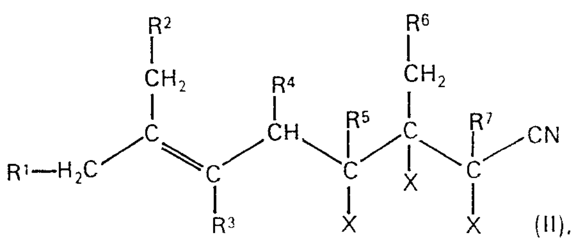

(II),

in der $R^1$ bis $R^7$ und die X die in Anspruch 1 angegebene Bedeutung haben, durch Behandeln mit verdünnten Mineralsäuren hydratisiert und gegebenenfalls anschliessend katalytisch hydriert.

3. Verfahren zur Herstellung der aliphatischen Methoxy- oder Hydroxynitrile der allgemeinen Formel I gemäss Anspruch 1, in der $R^7$ für -H steht, dadurch gekennzeichnet, dass man die entsprechenden Methoxy- bzw. Hydroxyketone der allgemeinen Formel III

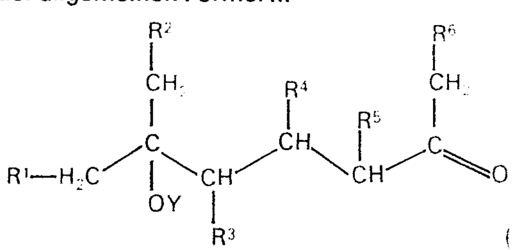

(III)

in der $R^1$ bis $R^6$ und Y die in Anspruch 1 angegebene Bedeutung haben, in Gegenwart eines schwachbasischen Katalysators in an sich bekannter Weise mit Cyanessigsäure umsetzt, wobei das sich bildende Kondensationsprodukt decarboxyliert wird, und gegebenenfalls anschliessend katalytisch hydriert.

4. Verwendung der gesättigten oder einfach ungesättigten aliphatischen Hydroxynitrile der allgemeinen Formel I gemäss Anspruch 1 als Duftstoff oder als Fixateure in Riechstoffkompositionen.

**Claims**

1. Saturated or mono-unsaturated aliphatic methoxynitriles and hydroxynitriles of the general formula I

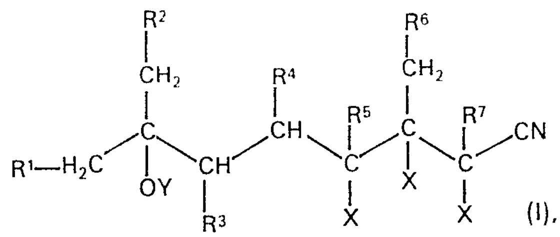

(I),

where $R^1$ to $R^7$ and Y are each H or $-CH_3$, and the X's are all H or adjacent pairs of X's denote a further bond between the carbon atoms bearing them, and the third X is -H.

2. A process for the production of the aliphatic hydroxynitriles of the general formula I as claimed in claim 1, characterized in that the corresponding nitriles of the general formula II

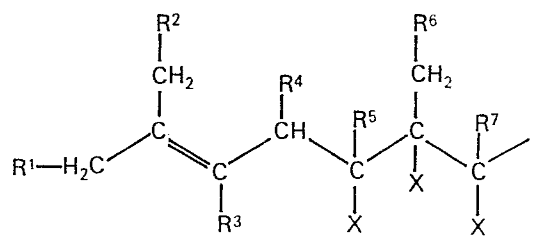

(II),

where $R^1$ to $R^7$ and the X's have the meanings given in claim 1 are hydrated by treatment with dilute mineral acids and, if desired, subsequently hydrogenated catalycally.

3. A process for the production of the aliphatic methoxynitriles or hydroxynitriles of the general formula I as claimed in claim 1, where $R^7$ is -H, characterized in that the corresponding methoxyketones or hydroxyketones of the general formula III

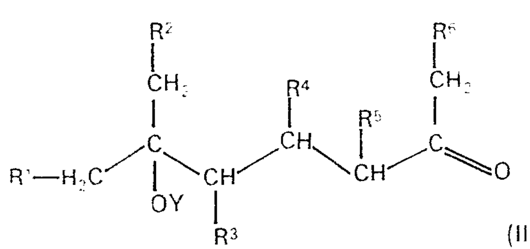

(III),

where $R^1$ to $R^6$ and Y have the meanings given in claim 1, are reacted with cyanoacetic acid in a conventional manner in the presence of a weakly

basic catalyst, the condensation product which forms being decarboxylated, and, if desired, subsequently hydrogenated catalytically.

4. Use of the saturated or mono-unsaturated aliphatic hydroxynitriles of the general formula I as claimed in claim 1 as odorants or as fixatives in perfume compositions.

**Revendications**

1. Méthoxy- et hydroxynitriles aliphatiques saturés ou à simple insaturation de formule générale I

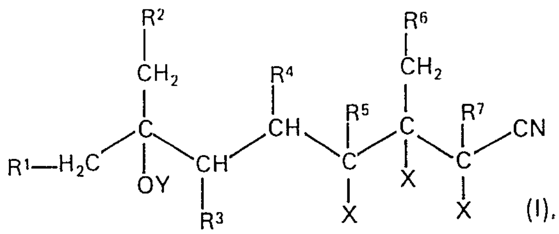

(I),

dans laquelle $R^1$ à $R^7$ et Y sont mis pour H ou $-CH_3$ et ou bien les symboles X représentent tous des atomes de H, ou bien deux X voisins sont mis pour une liaison supplémentaire entre les atomes de C qui les portent et le troisième X représente -H.

2. Procédé pour la préparation des hydroxynitriles aliphatiques de formule générale I selon la revendication 1, caractérisé en ce qu'on hydrate, par traitement par des acides minéraux dilués, les nitriles correspondants de formule générale II

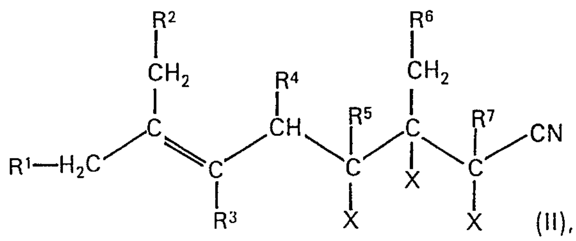

(II),

dans laquelle $R^1$ à $R^7$ et les symboles X ont les significations données la revendication 1, et, le cas échéant, on procède immédiatement après à une hydrogénation catalytique.

3. Procédé pour la préparation des méthoxy- ou hydroxynitriles aliphatiques de formule I selon la revendication 1, dans laquelle $R^7$ est mis pour -H, caractérisé en ce qu'on fait réagir les méthoxy- ou hydroxycétones correspondantes de formulé générale III

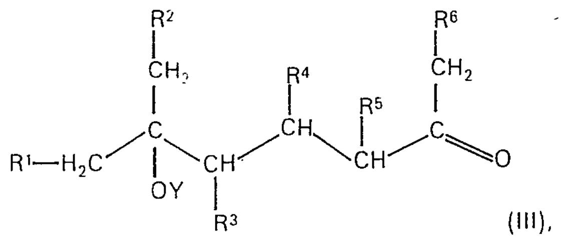

(III),

dans laquelle $R^1$ à $R^6$ et Y ont les significations données dans la revendication 1, avec l'acide cyanacétique en présence d'un catalyseur faiblement basique de façon connue en soi, le produit de condensation qui se forme étant décarboxylé et, le cas échéant, soumis immédiatement après à une hydrogénation catalytique.

4. Utilisation des hydroxynitriles aliphatiques saturés ou à simple insaturation de formule générale I selon la revendication 1, en tant que matières odorantes ou en tant que fixateurs dans des compositions de parfums.